# EUROPÄISCHE PATENTSCHRIFT

(11) **EP 1 882 457 B1**
(45) Veröffentlichungstag und Bekanntmachung des Hinweises auf die Patenterteilung: **28.03.2012**
(21) Anmeldenummer: 06015447.3
(22) Anmeldetag: 25.07.2006
(51) Int. Cl.: A61B 19/00, A61F 2/46

(54) **Verfahren und Vorrichtung zur Darstellung von Objektausrichtung an Kugelgelenken**
Method and device for representing the orientation of an object to ball joints
Procédé et dispositif destinés à la représentation d'orientation d'un objet aux joints sphériques

(43) Veröffentlichungstag der Anmeldung: 30.01.2008
(73) Patentinhaber: BrainLAB AG, 85622 Feldkirchen (DE)
(72) Erfinder: Dick, Robert, 80686 München (DE)
(74) Vertreter: Rögner, Jürgen

(56) Entgegenhaltungen:
- WO-A-02/080824
- WO-A-03/096920
- US-A1- 2003 105 470
- US-A1- 2004 152 970
- US-A1- 2004 267 121
- US-A1- 2006 095 047

## Beschreibung

Die Erfindung betrifft die Darstellung von Objektausrichtungen an Kugelgelenken, insbesondere betrifft sie ein Verfahren zur medizintechnischen Darstellung der Ausrichtung eines Objekts an einem Kugelgelenk sowie eine Vorrichtung zur Durchführung eines solchen Verfahrens.

Wenn in der Medizin Kugelgelenk-Implantate gesetzt werden, insbesondere beim Einsetzen von Pfannenimplantaten für Hüftgelenke, kann diese Prozedur durch eine medizintechnische Navigation unterstützt werden. Eine solche medizintechnische Navigation erlaubt es, die Positionen des Implantat bzw. des Patienten und die Relativpositionen zu bestimmen und zu verfolgen und dem behandelnden Arzt über eine Bildschirmausgabe visuelle Unterstützung bei der Positionierung des Implantates zu geben.

Derzeit wird die Position eine Hüftimplantats durch zwei Winkel beschrieben, die dann entsprechend auch im Rahmen von Navigationsdaten als Hilfe für den Chirurgen ausgegeben werden. Diese beiden Winkel sind die Inklination und die Anteversion, und die Abweichung von einer Position zu einer anderen wird durch eine Anteversionsänderung und durch eine Inklinationsänderung beschrieben. Eine übliche Definition für die Anteversion ist die Winkelbewegung beim Vorheben des Beines; beim Zurückführen des Beines wird die Gegenrichtung als Retroversion bezeichnet. Die Inklination und die Deklination sind hierzu senkrechte und einander entgegengesetzte Bewegungsrichtungen. Entsprechendes gilt beispielsweise für die Bewegung des Oberarms im Schulter-Kugelgelenk.

Problematisch ist hierbei einerseits, dass in der medizinischen Literatur unterschiedliche Arten gibt, die Inklination und die Anteversion zu berechnen.

Hierdurch können Missverständnisse entstehen, die bei medizinischen Eingriffen zur Positionierung eines Implantates nicht tolerierbar sind. Andererseits ist die Ausgabe von zwei Winkeln, nämlich jeweils der Winkel für die Anteversion und die Inklination nicht besonders anschaulich, was Positionskorrekturen erschwert, insbesondere wenn der behandelnde Arzt nicht über eine weitreichende Erfahrung verfügt.

Aus der WO-A-02/080824 ist eine Vorrichtung und ein Verfahren bekannt, welche im Zuge der Implantation eines künstlichen Hüftgelenk zur Ermittlung funktionsbestimmender geometrischer Größen des Gelenks dienen. Das Dokument offenbart insbesondere ein Verfahren zur medizintechnischen graphischen Darstellung der aktuellen Ausrichtung eines Objekts, z.B. einer Implantathalte- und -Setzvorrichtung, an einem Kugelgelenk, wobei die Abweichung der Vorzugsrichtung des Objekts zu einer Soll- oder Nullwinkelrichtung mittels eines Fadenkreuzes in einem Koordinatensystem angezeigt wird. Dabei ist die Anteversion bzw. die Retroversion auf der Abszisse und die Abduktion bzw. die Adduktion auf der Ordinate aufgetragen. Die Soll- oder Nullwinkelrichtung des Objekts ist dann erreicht, wenn das die Ist-Ausrichtung darstellende Fadenkreuz im Ursprung des Koordinatensystems zu liegen kommt.

Es ist die Aufgabe der vorliegenden Erfindung, die medizintechnische Darstellung von Objektausrichtungen an Kugelgelenken zu optimieren. Insbesondere soll eine unmissverständliche Darstellung ermöglicht werden, die eine einfache und anschauliche Positionswiedergabe bzw. Positionskorrektur für das Objekt gestattet.

Diese Aufgabe wird erfindungsgemäß durch ein Verfahren gemäß dem Anspruch 1 sowie eine Vorrichtung gemäß dem Anspruch 10 gelöst. Die Unteransprüche definieren bevorzugte Ausführungsformen der Erfindung.

Die Erfindung hat also ein Verfahren zur medizintechnischen Darstellung der Ausrichtung eines Objektes an einem Kugelgelenk zum Gegenstand, bei dem
- am Kugelgelenk eine aus der Gelenkpfanne heraus ragende Nullwinkeleinrichtung definiert wird, und Richtungen definiert werden, die als anatomische Richtungen definiert sind,
- das Objekt eine definierte Vorzugsrichtung aufweist, die im Verhältnis zum Nullwinkel auszurichten ist, und bei dem
- eine graphische Darstellung der Objektausrichtung erfolgt, bei welcher die Abweichung der Vorzugsrichtung zurn Nullwinkelerichtung zahlenmäßig dargestellt wird und das Ausrichtungsverhältnis der Vorzugsrichtung zu den anatomischen Richtungen auf einer Umfangsdarstellung als Anteilswert der anatomischen Richtungen dargestellt wird.

Der Chirurg braucht eine eindeutige und einfach verständliche Information darüber, wie die Implantatposition verändert ist, beispielsweise gegenüber seiner ursprünglichen Planung. Das erfindungsgemäße Verfahren trennt diese benötigte Information in eine Winkelabweichung (Vorzugsrichtung/Nullwinkel) und eine Richtungsinformation (Ausrichtungsverhältnis der Vorzugsrichtung zu den anatomischen Richtungen). Weil die Winkelinformation unabhängig von der Richtungsinformation dargestellt wird, ist sie eindeutig und unmissverständlich, und die Darstellung der Richtungsinformation als Umfangsdarstellung mit Anteilswerten von anatomischen Richtungen macht die entsprechende Abweichung sehr gut sichtbar und verständlich. Der Chirurg weiß beim Betrachten der erfindungsgemäßen Darstellung exakt, wie groß seine Abweichung als Betrag (in Winkelgraden) ist und in welche Richtung sie geht. Mit einer solchen visuellen Unterstützung lässt sich eine Abweichung auch in einfacher Weise korrigieren, indem eine entsprechende Winkelverstellung in exakter Gegenrichtung vorgenommen wird.

Bei einer bevorzugten Ausführungsform sind auch die anatomischen Richtungen Winkelrichtungen, wie es sich für Kugelgelenke am menschlichen Körper anbietet.

Die Vorzugsrichtung des Objekts kann durch eine Achse oder eine Ebene des Objekts definiert werden. Es ist aber auch möglich, sie durch eine Achse oder durch eine Ebene eines an dem Objekt angebrachten Gegenstandes zu definieren, beispielsweise einer Halterung für das Objekt.

Bei einer speziellen Ausführungsvariante ist das Objekt ein Implantat, insbesondere ein Gelenkpfannenimplantat, und die Vorzugsrichtung ist die Axialrichtung der Implantat-Halte- und -Setzvorrichtung.

Die vorab genannten anatomischen Richtungen können je nach Eignung sämtliche bekannten und üblichen anatomischen Richtungen sein. Im Folgenden wird eine Auswahl angegeben:
- Anteversion bzw. Retroversion,
- Inklination bzw. Deklination,
- Abduktion bzw. Adduktion,
- Eversion bzw. Inversion,
- Rotation als Winkelabweichung von der anatomischen Normalstellung, insbesondere vom Nullwinkel,
- Elevation,
- transversal, sagittal, longitudinal, frontal, medial, lateral.

Ein erfindungsgemäßes Verfahren kann so ausgestaltet sein, dass die Umfangsdarstellung eine Abbildung eines geschlossenen Umfangs, insbesondere eines Kreis- oder Ellipsenumfangs ist (auch eckige Umfänge sind möglich), der durch zwei senkrecht aufeinander stehende anatomische Anzeigen, insbesondere Hauptrichtungsanzeigen unterteilt ist, wobei eine Zeigereinrichtung den Anteilswert der aktuellen anatomischen Winkelrichtung für das Objekt darstellt. Die genannte Zeigereinrichtung kann alle möglichen Formen umfassen, beispielsweise die eines tatsächlichen Zeigers oder Pfeilzeigers, aber auch die Form von Balken (auch farbigen Balken), die durch eine Abgrenzung separiert sind, deren Position sich je nach Lage des Objekts ändert.

Die Erfindung betrifft auch ein Programm, das, wenn es auf einem Computer läuft oder in einem Computer geladen ist, den Computer veranlasst, ein Verfahren durchzuführen, wie es oben in unterschiedlichen Ausführungsformen beschrieben worden ist. Sie hat auch ein Computer-Speichermedium zum Gegenstand, das ein solches Programm aufweist.

Gemäß einem weiteren Aspekt betrifft die vorliegende Erfindung eine medizintechnische Darstellungsvorrichtung für die Darstellung der Ausrichtung eines Objekts an einem Kugelgelenkt, mit
- einer Datenspeicherungs- und Datenverarbeitungseinrichtung, die anatomische Daten speichert, mittels der am Kugelgelenk eine aus der Gelenkpfanne heraus ragende Nullwinkelerichtung definiert wird, und Richtungen definiert werden, die als anatomische Richtungen definiert sind, und mittels derer eine Objekt-Vorzugsrichtung definiert wird, die im Verhältnis zur Nullwinkelerichtung auszurichten ist, und mit
- einer Graphik-Verarbeitungseinheit und einer Graphikausgabe, mittels der eine graphische Darstellung der Objektausrichtung erfolgt, bei welcher die Abweichung der Vorzugsrichtung zun Nullwinkel zahlenmäßig dargestellt wird und das Ausrichtungsverhältnis der Vorzugsrichtung zu den anatomischen Richtungen auf einer Umfangsdarstellung als Anteilswert der anatomischen Richtungen dargestellt wird.

Die Erfindung wird im Weiteren anhand bevorzugter Ausführungsbeispiele erläutert. Sie kann alle hierin beschriebenen Merkmale einzeln sowie in jedweder sinnvollen Kombination umfassen, wobei die Verfahrensmerkmale auch vorrichtungsmäßig umgesetzt werden können. In den beiliegenden Zeichnungen zeigen:
- Figur 1: eine Aufsicht auf eine Gelenkpfanne mit einer Darstellung der anatomischen Richtungen;
- Figur 2: eine erfindungsgemäße Darstellung einer Ausrichtungsabweichung für ein Hüftgelenkimplantat;
- Figur 3: eine Darstellung gemäß Figur 2 mit einer anderen Ausrichtungsabweichung.

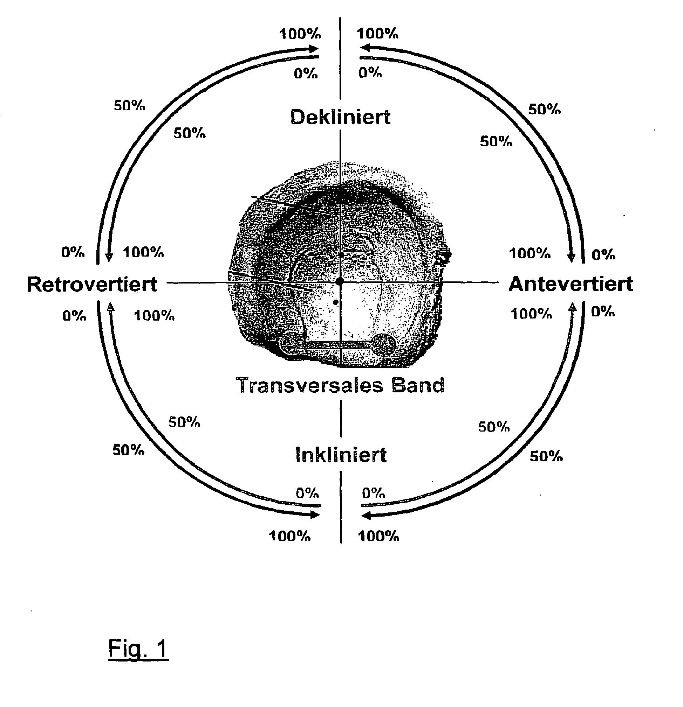

Die Figur 1 macht deutlich, wie am Beispiel einer Hüftgelenkpfanne, die mit einem medizintechnischen Navigations- bzw. Trackingsystem geartet bzw. verfolgt (positionsmäßig erfasst) wird, die anatomischen Richtungen definiert werden, die für das erfindungsgemäße Darstellungsverfahren benötigt werden. Die beiden Hauptrichtungen sind die Anteversion und die Inklination, wobei die Anteversion als Gegenrichtung die Retroversion und die Inklination als Gegenrichtung die Deklination hat. Diese beiden Richtungen stehen senkrecht aufeinander, und in der umfänglichen Darstellung, bei der jeweils in den Quadranten Anteilswerte von 0 bis 100% angegeben wird, ist für jede Stelle am Umfang ein bestimmter Wert für Anteversion, Retroversion, Inklination und Deklination zuordnungsfähig. Die Richtungsinformation wird in der Ursprungsebene oder senkrecht zur Ursprungsebene entlang der gezeigten anatomischen Richtungen darstellbar. Das transversale Band wird im vorliegenden Fall verwendet, um die Hauptrichtungen Anteversion und Inklination zu definieren, wobei die Anteversion parallel zum transversalen Band liegt und die Inklination senkrecht dazu. Man könnte natürlich auch andere Definitionen für die Anteversion und die Inklination wählen.

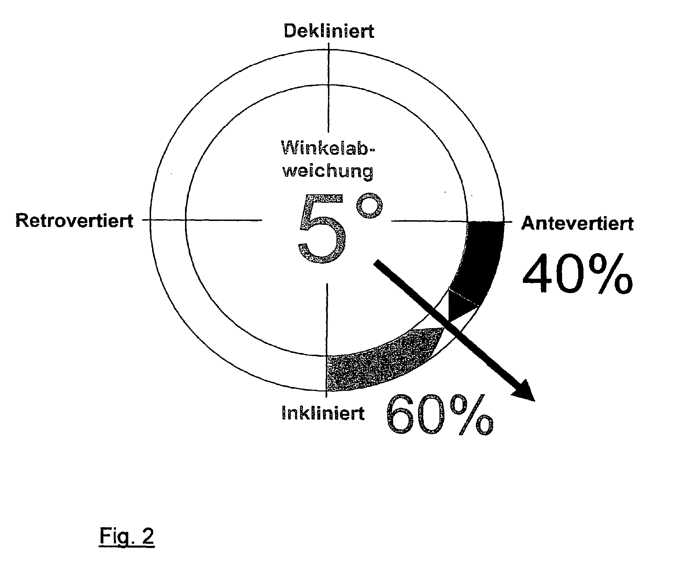

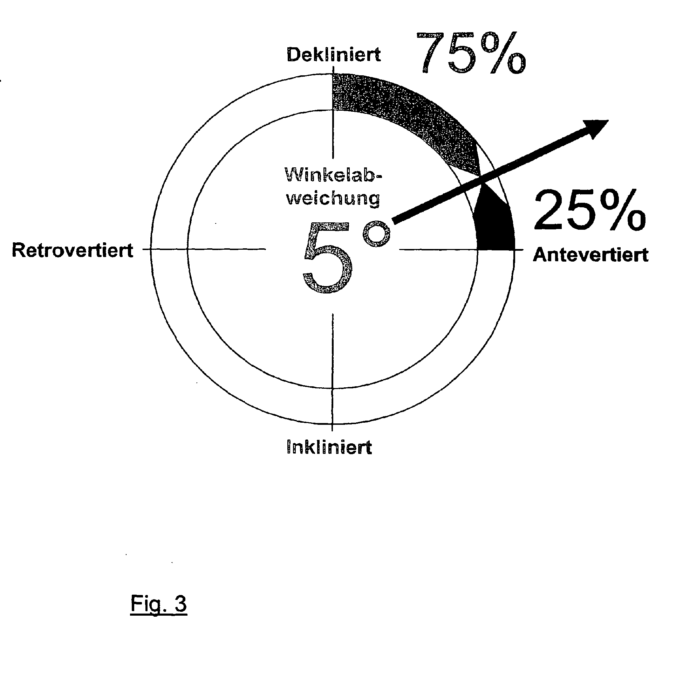

Anstelle der üblichen Information des Chirurgen über lediglich zwei Winkelabweichungen in nicht eindeutig berechneten Winkelrichtungen, nämlich lediglich mit Inklinations- und Anteverstionsabweichungen, wird die Abweichungsinformation gemäß der vorliegenden Erfindung in zwei Informationen aufgespaltet, nämlich in eine absolute Winkelabweichung und eine Richtungsinformation. Die so aufgespaltete Information wird auch separat dargestellt, wobei die Winkelabweichung direkt ermittelt wird, entweder von einer Ebene zu einer anderen oder von einem Vektor zu einem anderen. Anhand der Figuren 2 und 3 wird das erfindungsgemäße Darstellungskonzept nunmehr verdeutlicht. Die Darstellung der Objekt-Ausrichtungsabweichung erfolgt, wie in der Figur 2 zu sehen ist, durch eine kreisförmige Umfangsdarstellung, in deren Mitte ein absoluter Wert für die Winkelabweichung angegeben wird. Man kann sich die Darstellung so vorstellen, als ob man, wie in Figur 1 auch die Gelenkpfanne sieht. Die in der Mitte separat dargestellte Winkelabweichung von 5° zeigt nun lediglich, dass das Objekt, hier beispielsweise eine Setzvorrichtung für das einzubringende Implantat, an der das Implantat gehalten wird, um 5° von der geplanten Richtung abweicht. Die Information darüber, in welche Richtung die Abweichung geht, wird durch die Umfangsdarstellung, das heißt den Kreisbalken, geliefert, und im Fall der Figur 2 ist die Richtung 60% inkliniert und 40% antevertiert. Der Chirurg weiß nun, dass er seinen Implantatträger (Setzvorrichtung) in exakt die Gegenrichtung bewegen muss, um die Inklinations- und Anteversionsabweichung abzuweichen, und zwar um nur einen geringen Winkel von 5%. Wenn er den ebenfalls medizintechnisch navigierten bzw. getrackten Implantatträger bewegt, wird sich auch die Grafik anpassen, und zwar so lange bis die Winkelabweichung mit 0% angegeben wird und kein Zeiger mehr zu sehen ist. Entsprechendes gilt für eine Abweichung, wie sie beispielsweise in der Figur 3 dargestellt ist, wo eine Deklination von 75% und eine Anteversion von 25% vorliegt.

Weitere Beispiele: Eine Abweichung von 100° in Anteversionsrichtung würde zu einem Pfeil führen, der horizontal zur rechten Seite zeigt, und 100% antevertiert würde mit dem jeweilig aktuellen Winkel angezeigt werden. Im Vergleich mit einem Kompass wäre dies eine Richtung, die genau nach "Osten" geht.

Es wäre grundsätzlich auch denkbar, den Pfeil je nach der Größe der Winkelabweichung länger oder kürzer zu machen, so dass hier eine separate Nullwinkel-Abweichungs-Information, insbesondere als zusätzliche Information bereitgestellt wird.

Durch diese anschauliche getrennte Widergabe von Winkelabweichung und Richtungsabweichung lassen sich diese Abweichungen schnell und gezielt korrigieren, und zwar auch von Chirurgen, die noch keine sehr weitgehende Erfahrung auf dem entsprechenden medizinischen Gebiet haben.

## Patentansprüche

1. Verfahren zur medizintechnischen Darstellung der Ausrichtung eines Objekts an einem Kugelgelenk, bei dem
- am Kugelgelenk eine aus der Gelenkpfanne heraus ragende Nullwinkelrichtung definiert wird, und Richtungen definiert werden, die als anatomische Richtungen definiert sind,
- das Objekt eine definierte Vorzugsrichtung aufweist, die im Verhältnis zur Nullwinkelrichtung auszurichten ist, und bei dem
- eine graphische Darstellung der Objektausrichtung erfolgt, bei welcher die Abweichung der Vorzugsrichtung zur Nullwinkelrichtung zahlenmäßig dargestellt wird und das Ausrichtungsverhältnis der Vorzugsrichtung zu den anatomischen Richtungen auf einer Umfangsdarstellung als Anteilswert der anatomischen Richtungen dargestellt wird.

2. Verfahren nach Anspruch 1, bei dem die anatomischen Richtungen Winkelrichtungen sind.

3. Verfahren nach Anspruch 1 oder 2, bei dem die Vorzugsrichtung des Objekts durch eine Achse oder durch eine Ebene des Objekts oder einer an ihm angebrachten Objekthalterung definiert wird.

4. Verfahren nach einem der Ansprüche 1 bis 3, bei dem das Objekt ein Implantat, insbesondere ein Gelenkpfannenimplantat ist und die Vorzugsrichtung die Axialrichtung der Implantat-Halte- und/oder Setzvorrichtung ist.

5. Verfahren nach einem der Ansprüche 1 bis 4, bei dem die anatomischen Richtungen eine oder mehrere der folgenden Richtungen umfassen:
- Anteversion bzw. Retroversion,
- Inklination bzw. Deklination,
- Abduktion bzw. Adduktion,
- Eversion bzw. Inversion,
- Rotation als Winkelabweichung von der anatomischen Normalstellung, insbesondere von der Nullwinkelrichtung,
- Elevation,
- transversal, sagittal, longitudinal, frontal, medial, lateral.

6. Verfahren nach einem der Ansprüche 1 bis 5, die Umfangsdarstellung eine Abbildung eines geschlossenen Umfangs, insbesondere eines Kreis- oder Ellipsenumfangs ist, der durch zwei senkrecht aufeinander stehende anatomische Richtungsanzeigen unterteilt ist, wobei eine Zeigereinrichtung den Anteilswert der anatomischen Richtungen für das Objekt darstellt.

7. Verfahren nach einem der Ansprüche 1 bis 6, bei dem die Nullwinkelrichtung anhand von anatomischen Merkmale der Gelenkpfanne bzw. der Gelenkpfannenumgebung bestimmt wird, insbesondere durch die Lage des transversalen Bandes.

8. Programm, das, wenn es auf einem Computer läuft oder in einem Computer geladen ist, den Computer veranlasst, ein Verfahren gemäß einem der Ansprüche 1 bis 7 durchzuführen.

9. Computerprogramm-Speichermedium, das ein Programm nach Anspruch 8 aufweist.

10. Medizintechnische Darstellungsvorrichtung für die Darstellung der Ausrichtung eines Objekts an einem Kugelgelenk, mit
- einer Datenspeicherungs- und Datenverarbeitungseinrichtung, die anatomische Daten speichert, mittels der am Kugelgelenk eine aus der Gelenkpfanne heraus ragende Nullwinkelrichtung definiert wird, und Richtungen definiert werden, die als anatomische Richtungen definiert sind, und mittels derer eine Objekt-Vorzugsrichtung definiert wird, die im Verhältnis zur Nullwinkelrichtung auszurichten ist, und mit
- einer Graphik-Verarbeitungseinheit und einer Graphikausgabe, mittels derer eine graphische Darstellung der Objektausrichtung erfolgt, bei welcher die Abweichung der Vorzugsrichtung zur Nullwinkelrichtung zahlenmäßig dargestellt wird und das Ausrichtungsverhältnis der Vorzugsrichtung zu den anatomischen Richtungen auf einer Umfangsdarstellung als Anteilswert der anatomischen Richtungen dargestellt wird.

## Claims

1. A method for displaying, in the field of medical technology, the orientation of an object on a ball joint, wherein:
- a zero angle direction projecting out of the joint cavity on the ball joint is defined, and directions are defined which are defined as anatomical directions;
- the object exhibits a defined preferred direction which is to be orientated in relation to the zero angle direction; and wherein
- the object orientation is graphically displayed, wherein the deviation between the preferred direction and the zero angle direction is displayed numerically, and the orientation relationship between the preferred direction and the anatomical directions is displayed on a circumferential representation as a proportional value of the anatomical directions.

2. The method according to claim 1, wherein the anatomical directions are angular directions.

3. The method according to claim 1 or 2, wherein the preferred direction of the object is defined by an axis or plane of the object or an axis or plane of an object holder attached to it.

4. The method according to any one of claims 1 to 3, wherein the object is an implant, in particular a joint cavity implant, and the preferred direction is the axial direction of the implant holding and/or placing device.

5. The method according to any one of claims 1 to 4, wherein the anatomical directions include one or more of the following directions:
- anteversion or retroversion;
- inclination or declination;
- abduction or adduction;
- eversion or inversion;
- rotation as an angular deviation from the anatomical normal position, in particular from the zero angle direction;
- elevation;
- transversal, sagittal, longitudinal, frontal, medial, lateral.

6. The method according to any one of claims 1 to 5, wherein the circumferential representation is an image of a closed circumference, in particular a circular or elliptical circumference, which is sub-divided by two perpendicularly superimposed anatomical directional displays, wherein a pointer means displays the proportional value of the anatomical directions for the object.

7. The method according to any one of claims 1 to 6, wherein the zero angle direction is determined on the basis of anatomical features of the joint cavity and/or the surrounding of the joint cavity, in particular by the position of the transversal ligament.

8. A program which, when it is running on a computer or is loaded onto a computer, causes the computer to perform a method in accordance with any one of claims 1 to 7.

9. A computer program storage medium comprising a program according to claim 8.

10. A medical displaying device for displaying the orientation of an object on a ball joint, comprising:
- a data storing and processing means which stores anatomical data, by means of which a zero angle direction projecting out of the joint cavity on the ball joint is defined, and directions are defined which are defined as anatomical directions, and by means of which a preferred direction of the object is defined which is to be orientated in relation to the zero angle direction; and comprising
- a graphic processing unit and a graphic output by means of which the object orientation is graphically displayed, wherein the deviation between the preferred direction and the zero angle direction is displayed numerically, and the orientation relationship between the preferred direction and the anatomical directions is displayed on a circumferential representation as a proportional value of the anatomical directions.

## Revendications

1. Procédé pour la représentation en technique médicale de l'orientation d'un objet sur une articulation rotoïde, selon lequel :
- une direction angulaire nulle, s'avançant hors de la cavité articulaire, est définie au niveau de l'articulation rotoïde, et sont définies des directions qui sont définies en tant que directions anatomiques,
- l'objet comporte une direction préférentielle définie, qui doit être orientée par rapport à la direction angulaire nulle, et selon lequel
- une représentation graphique de l'orientation de l'objet est effectuée, sur laquelle est représentée en chiffres la divergence de la direction préférentielle par rapport à la direction angulaire nulle, et le rapport de l'orientation de la direction préférentielle par rapport aux directions anatomiques est représenté sur une représentation périphérique en tant que valeur proportionnelle des directions anatomiques.

2. Procédé selon la revendication 1, selon lequel les directions anatomiques sont des directions angulaires.

3. Procédé selon la revendication 1 ou 2, selon lequel la direction préférentielle de l'objet est définie par un axe ou par un plan de l'objet ou d'un support d'objet monté sur celui-ci.

4. Procédé selon l'une quelconque des revendications 1 à 3, selon lequel l'objet est un implant, en particulier un implant de cavité articulaire, et la direction préférentielle est la direction axiale du dispositif de fixation et/ou de mise en place de l'implant.

5. Procédé selon l'une quelconque des revendications 1 à 4, selon lequel les directions anatomiques comprennent une ou plusieurs des directions suivantes :
- antéversion ou rétroversion,
- inclinaison ou déclinaison,
- abduction ou adduction,
- éversion ou inversion,
- rotation en tant que divergence angulaire par rapport à la position normale anatomique, en particulier par rapport à la direction angulaire nulle,
- élévation,
- transversale, sagittale, longitudinale, frontale, médiane, latérale.

6. Procédé selon l'une quelconque des revendications 1 à 5, selon lequel la représentation périphérique est une reproduction d'un périmètre fermé, en particulier la périphérie d'un cercle ou d'une ellipse, qui est divisée par deux indications de directions anatomiques perpendiculaires l'une à l'autre, un dispositif indicateur représentant la valeur proportionnelle des directions anatomiques pour l'objet.

7. Procédé selon l'une quelconque des revendications 1 à 6, selon lequel la direction angulaire nulle est déterminée à l'appui de caractéristiques anatomiques de la cavité articulaire ou de l'environnement de la cavité articulaire, en particulier par la longueur du ligament transversal.

8. Programme qui, pendant son déroulement sur un ordinateur ou pendant son chargement dans un ordinateur, ordonne à l'ordinateur de mettre en oeuvre un procédé selon l'une quelconque des revendications 1 à 7.

9. Support de mémoire pour programme informatique, qui contient un programme selon la revendication 8.

10. Dispositif de représentation en technique médicale pour la représentation de l'orientation d'un objet au niveau d'une articulation rotoïde, comportant
- un dispositif de stockage des données et de traitement des données, qui stocke des données anatomiques, au moyen desquelles une direction angulaire nulle, s'avançant hors de la cavité articulaire, est définie au niveau de l'articulation rotoïde et sont définies des directions qui sont définies en tant que directions anatomiques, et au moyen desquelles est définie une direction préférentielle de l'objet, qui doit être orientée par rapport à la direction angulaire nulle, et comportant
- une unité de représentation graphique et un système d'édition graphique, au moyen desquels est effectuée une représentation graphique de l'orientation de l'objet, pendant laquelle est représentée en chiffres la divergence de la direction préférentielle par rapport à la direction angulaire nulle, et le rapport de l'orientation de la direction préférentielle par rapport aux directions anatomiques est représenté sur une représentation périphérique en tant que valeur proportionnelle des directions anatomiques.
